# EUROPEAN PATENT SPECIFICATION

(11) **EP 3 985 101 B1**
(45) Date of publication and mention of the grant of the patent: **23.04.2025**
(21) Application number: 20834965.4
(22) Date of filing: 03.07.2020
(51) Int. Cl.: C12N 5/02, C12N 5/00, A61K 35/12

(54) **METHOD FOR PRODUCING CULTURE MEDIUM COMPOSITION FOR SUSPENSION CULTURING ADHERENT CELLS**
VERFAHREN ZUR HERSTELLUNG EINER KULTURMEDIUMZUSAMMENSETZUNG ZUR SUSPENSION VON ADHÄRENTEN ZELLEN
PROCÉDÉ DE PRODUCTION D'UNE COMPOSITION DE MILIEU DE CULTURE POUR LA CULTURE EN SUSPENSION DE CELLULES ADHÉRENTES

(30) Priority: 04.07.2019 JP 2019125536
(43) Date of publication of application: 20.04.2022
(73) Proprietor: Nissan Chemical Corporation, Tokyo 103-6119 (JP)
(72) Inventor: HATANAKA, Daisuke, Funabashi-shi, Chiba 274-0052 (JP); KANAKI, Tatsuro, Tokyo, 103-6119 (JP); HAYASHI, Hisato, Tokyo 103-6119 (JP)
(74) Representative: J A Kemp LLP
(86) International application number: PCT/JP2020/026130
(87) International publication number: WO 2021/002448

(56) References cited:
- WO-A1-2012/119012
- WO-A1-2015/111686
- JP-A- 2007 319 074
- JP-A- 2011 167 237
- NOH H K ET AL: "Electrospinning of chitin nanofibers: Degradation behavior and cellular response to normal human keratinocytes and fibroblasts", BIOMATERIALS, ELSEVIER, AMSTERDAM, NL, vol. 27, no. 21, 1 July 2006 (2006-07-01), pages 3934 - 3944, XP027951336, ISSN: 0142-9612, [retrieved on 20060701]
- YAN-YAN WANG ET AL: "Cellular compatibility of RGD-modified chitosan nanofibers with aligned or random orientation", BIOMEDICAL MATERIALS, INSTITUTE OF PHYSICS PUBLISHING, BRISTOL, GB, vol. 5, no. 5, 1 October 2010 (2010-10-01), pages 54112, XP020184956, ISSN: 1748-605X, DOI: 10.1088/1748-6041/5/5/054112
- HASSANZADEH, P. ET AL.: "Chitin nanofiber micropatterned flexible substrates for tissue engineering", JOURNAL OF MATERIALS CHEMISTRY B, vol. 1, no. 34, 14 September 2013 (2013-09-14), pages 4217 - 4224, XP055214698, DOI: 10.1039/c3tb20782j
- ALI HUSSAIN; COLLINS GEORGE; YIP DEREK; CHO CHEUL H: "Functional 3-D Cardiac Co-Culture Model Using Bioactive Chitosan Nanofiber Scaffolds", BIOTECHNOLOGY AND BIOENGINEERING, vol. 110, no. 2, 5 October 2012 (2012-10-05), pages 637 - 647, XP055214701

## Description

### [Technical Field]

The present invention relates to a production method of a medium composition for suspension culture of adherent cells, as well as a related composition for addition to a medium and a related medium composition.

### [Background Art]

In recent years, methods for transplanting or injecting cells into a living body have been developed mainly in the fields of medicine and beauty. Among them, somatic stem cells and progenitor cells are attracting attention because they have a lower risk of canceration, a shorter differentiation period, and the like compared with pluripotent stem cells.

When these cells are utilized, a large number of the cells in good condition need to be provided. As a method therefor, a method including culturing and proliferating stem cells and the like while being adhered to a microcarrier and the like is known.

However, currently available microcarriers form sediment in the culture medium under static conditions, and need to be stirred during culture. A problem has been pointed out that cell death occurs due to collision between micro carriers during the stirring. In addition, the efficiency of cell proliferation is not sufficient, and further improvement is expected.

The present inventors have developed a medium composition for culturing animal and plant cells and/or tissues in a suspended state by using a nanofiber of polysaccharides and the like having enhanced dispersibility in water (patent document 1) .

Furthermore, the present inventors have found that nanofibers composed of water-insoluble polysaccharides can be used as a common carrier in various operations such as i) suspension culture, ii) differentiation induction, iii) transportation and preservation under non-freezing conditions, iv) transplantation, v) recovery of bioactive substance from culture supernatant and the like of adherent cells (patent document 2, patent document 3).

### [Document List]

patent document 1: WO 2015/111686
patent document 2: WO 2017/175751
patent document 3: WO 2018/182016
JP2011167237A is titled: bio-applicable material.
Noh et al. Biomaterials 2006, vol. 27, no. 21, 3934-3944 is titled: electrospinning of chitin nanofibers: degradation behavior and cellular response to normal human keratinocytes and fibroblasts.
WO2012119012A1 is titled: system and method for vascularized biomimetic 3-d tissue models.
Wang et al., Biomed. Mater. 2010, 5, 54112 is titled: cellular compatibility of RGD-modified chitosan nanofibers with aligned or random orientation.

### [Summary of Invention]

### [Technical Problem]

The present invention aims to provide a technique leading to large-scale production of adherent cells such as somatic stem cells, progenitor cells, and the like.

### [Solution to Problem]

The present inventors have conducted intensive studies in an attempt to solve the above-mentioned problems and found that proliferation of adherent cells can be promoted extremely efficiently by suspension culturing the adherent cells by using, as a carrier substrate, a nanofiber composed of water-insoluble polysaccharides and carrying an extracellular matrix. In addition, they have also found that the adherent cells can be suspension cultured in a stationary state by mixing nanofibers carrying an extracellular matrix in a liquid medium and culturing the cells while being adhered to the nanofibers, that expansion culture can be performed by simply adding a fresh medium composition without performing cell detachment treatment with trypsin or the like. Based on these findings, the present inventors have conducted further studies and completed the present invention.

Accordingly, the present invention provides the following.
[1] A method for producing a medium composition for suspension culture of an adherent cell, comprising the following steps:
   (i) a step of making vitronectin carried on a nanofiber composed of chitin,
   (ii) a step of adding the vitronectin-carrying chitin nanofiber obtained in step (i) to a medium, wherein carried is a state in which the nanofiber and vitronectin are attached or adsorbed without a chemical covalent bond.
[2] The method of [1], wherein the nanofiber carries 0.01 - 50 mg of vitronectin per 1 g of the nanofiber.
[3] The method of [1] or [2], wherein a chitosan nanofiber is further added in step (ii).
[4] The method of [3], wherein a content ratio (weight) of the chitin nanofiber carrying vitronectin and the chitosan nanofiber is chitin nanofiber carrying vitronectin:chitosan nanofiber = 1:0.5 - 20.
[5] A composition for addition to a medium, comprising a chitin nanofiber carrying vitronectin and a chitosan nanofiber, wherein carrying is a state in which the nanofiber and vitronectin are attached or adsorbed without a chemical covalent bond.
[6] The composition of [5], wherein the chitin nanofiber carries 0.01 - 50 mg of vitronectin per 1 g of the chitin nanofiber.
[7] The composition of any of [9] to [11], wherein a content ratio (weight) of the chitin nanofiber carrying vitronectin and the chitosan nanofiber contained in medium composition is chitin nanofiber carrying vitronectin:chitosan nanofiber = 1:0.5 - 20.
[8] A medium composition for suspension culture of an adherent cell, comprising the composition of any of [5] to [7].

### [Advantageous Effects of Invention]

According to the present invention, adherent cells can be suspension cultured in a stationary state by culturing them while being attached to nanofibers composed of water-insoluble polysaccharides and carrying extracellular matrix, without an operation such as shaking, rotation and the like. In addition, cell proliferation can be promoted by the effect of the extracellular matrix.

According to the present invention, maintenance culture of adherent cells can be performed or they may also be proliferated while maintaining the characters thereof.

### [Brief Description of Drawings]

Fig. 1 shows a phase-contrast micrograph of a chitin nanofiber aqueous dispersion after addition of a CBB staining solution.
Fig. 2 shows a phase-contrast micrograph of a chitin nanofiber/chitosan nanofiber aqueous dispersion after addition of a CBB staining solution.
Fig. 3 shows a phase-contrast micrograph of a vitronectin-carrying chitin nanofiber aqueous dispersion after addition of a CBB staining solution.
Fig. 4 shows a phase-contrast micrograph of a vitronectin-carrying chitin nanofiber/chitosan nanofiber aqueous dispersion after addition of a CBB staining solution.

### [Description of Embodiments]

The present invention is described in detail in the following.

### 1. Production method of medium composition

The present invention provides a production method of a medium composition for suspension culture of an adherent cell, comprising the following steps (hereinafter sometimes to be referred to as "the production method of the present invention", etc.):
(i) a step of making vitronectin carried on a nanofiber composed of chitin,
(ii) a step of adding vitronectin-carrying nanofiber obtained in step (i) to a medium,
wherein carried is a state in which the nanofiber and vitronectin are attached or adsorbed without a chemical covalent bond.

In the production method of the present invention, adherent cell is a cell that requires a scaffold such as a container wall and the like for survival and proliferation.

In the production method of the present invention, the adherent cell to be used in the present invention is not particularly limited and, for example, stem cell, progenitor cell, somatic non-stem cell, primary cultured cell, cell line, cancer cell and the like can be mentioned. Stem cell is a cell concurrently having an ability to replicate itself, and an ability to differentiate into other plural lineages. Examples of the adherent stem cell include, but are not limited to, somatic stem cell and the like such as mesenchymal stem cell, neural stem cell, hematopoietic stem cell, liver stem cell, pancreas stem cell, muscle stem cell, germ stem cell, intestinal stem cell, cancer stem cell, hair follicle stem cell and the like. Mesenchymal stem cell is a stem cell having differentiation potency into all or some of osteocyte, chondrocyte and adipocyte. Mesenchymal stem cell is present in a tissue such as bone marrow, peripheral blood, cord blood, adipose tissue and the like at a low frequency and can be isolated from these tissues by a known method. Progenitor cell is a cell on the way to differentiate from the aforementioned stem cell into a particular somatic cell or reproductive cell. Examples of the adherent progenitor cell include, but are not limited to, pre-adipocyte, cardiac muscle progenitor cell, endothelial progenitor cell, neural progenitor cell, liver progenitor cell, pancreas progenitor cell, kidney progenitor cell and the like. Examples of the adherent somatic non-stem cell include, but are not limited to, fibroblast, osteocyte, bone pericyte, keratinocyte, adipocyte, mesenchymal cell, epithelial cell, epidermal cell, endothelial cell, vascular endothelial cell, hepatocyte, chondrocyte, cumulus cell, neural cell, glial cell, neuron, oligodendrocyte, microglia, astrocyte, heart cell, esophagus cell, muscle cell (e.g., smooth muscle cell or skeletal muscle cell), pancreas beta cell, melanin cell, and the like. Primary cultured cell is a cell after separation of cells and tissues from a living body and in a state of culture before performing the first passage. The primary cultured cell may be a cell collected from any tissue, for example, skin, kidney, spleen, adrenal gland, liver, lung, ovary, pancreas, uterus, stomach, colon, small intestine, large intestine, bladder, prostate, testis, thymus, muscle, bond tissue, bone, joints, blood vessel tissue, blood, heart, eye, brain, nerve tissue and the like. Cell lines are cells that have acquired infinite proliferative capacity by an artificial operation in vitro. The adherent cell in the production method of the present invention is preferably a stem cell or progenitor cell, more preferably a mesenchymal stem cell.

The derivation of the adherent cell in the production method of the present invention is not particularly limited, and the cell may be derived from any animal or plant. Examples of the animal include insect, fish, amphibian, reptiles, birds, pancrustacea, hexapoda, mammals and the like, with preference given to mammal. Examples of the mammal include, but are not limited to, rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like. The plant is not particularly limited as long as the collected cells can be applied to liquid culture. Examples thereof include, but are not limited to, plants (e.g., ginseng, periwinkle, henbane, coptis, belladonna etc.) producing crude drugs (e.g., saponin, alkaloids, berberine, scopolin, phytosterol etc.), plants (e.g., blueberry, safflower, madder, saffron etc.) producing dye or polysaccharide (e.g., anthocyanin, safflower dye, madder dye, saffron dye, flavones etc.) to be a starting material for cosmetic or food, or plants producing a pharmaceutical active pharmaceutical ingredient, and the like. The medium produced by the production method of the present invention is, preferably, used for mammalian adherent cells.

In the present specification, nanofiber refers to a fiber having an average fiber diameter (D) of 0.001 to 1.00 µm. The average fiber diameter of the nanofiber to be used in the present invention is preferably 0.005 to 0.50 µm, more preferably 0.01 to 0.05 µm, further preferably 0.01 to 0.02 µm.

In the production method of the present invention, the aspect ratio (L/D) of the nanofiber to be used is not particularly limited and is obtained from average fiber length/average fiber diameter, and is generally 2 - 500, preferably 5 - 300, more preferably 10 - 250.

In the present specification, the average fiber diameter (D) of the nanofiber is determined as follows. First, a hydrophilizing treatment of a collodion support film manufactured by Okenshoji Co., Ltd. is performed for 3 min by an ion cleaner (JIC-410) manufactured by JEOL Ltd., several drops of a nanofiber dispersion (diluted with ultrapure water) to be the evaluation target is added dropwise, and dried at room temperature. This is observed under a transmission electron microscope (TEM, H-8000) (10,000-fold) manufactured by Hitachi, Ltd. at an accelerating voltage 200 kV. Using the obtained image, the fiber diameter of each one of the nanofibers (specimen number: 200 - 250) is measured, and the mean thereof is taken as the average fiber diameter (D).

In addition, the average fiber length (L) is determined as follows. A nanofiber dispersion to be the evaluation target is diluted to 100 ppm with pure water, and nanofibers are uniformly dispersed using an ultrasonic cleaner. The nanofiber dispersion is cast on a silicon wafer subjected in advance to a hydrophilizing treatment of the surface with conc. sulfuric acid, dried at 110°C for 1 hr and used as a sample. Using an image obtained by observing the obtained sample under a scanning electron microscope (SEM, JSM-7400F) (2,000-fold), the fiber length of each one of the nanofibers (specimen number: 150 - 250) is measured, and the mean thereof is taken as the average fiber length (L).

In a preferable embodiment, the nanofiber is, upon mixing with a liquid medium, uniformly dispersed in the liquid while maintaining the primary fiber diameter, substantially retains the cells attached to the nanofiber without substantially increasing the viscosity of the liquid, and shows an effect of preventing sediment thereof.

The nanofiber to be used in the production method of the present invention is constituted of water-insoluble polysaccharides, wherein the water-insoluble polysaccharides are chitin. Saccharides mean glycopolymers wherein not less than 10 single saccharides (e.g., triose, tetrose, pentose, hexsauce, heptose etc.) are polymerized.

Examples of water-insoluble polysaccharides include celluloses such as cellulose, hemicellulose and the like; chitinous substances such as chitin, chitosan and the like, and the like.. In the present specification, the "nanofiber composed of chitin" is sometimes referred to as "chitin nanofiber". The same applies to other water-insoluble polysaccharides.

The chitinous substance refers to one or more carbohydrates selected from the group consisting of chitin and chitosan. Major sugar units constituting chitin and chitosan are N-acetylglucosamine and glucosamine, respectively. Generally, chitin has a high N-acetylglucosamine content and is poorly soluble in acidic aqueous solution, and chitosan has a high glucosamine content and is soluble in acidic aqueous solution. For convenience, chitin contains not less than 50% of N-acetylglucosamine in the constituent sugar, and chitosan contains less than 50% of N-acetylglucosamine in the present specification.

As the starting material of chitin, many biological resources such as shrimps, crabs, insect, shells, mushrooms and the like can be used. The chitin to be used in the present invention may be one having α-form crystal structure such as chitin derived from crab shell, shrimp shell and the like, or one having β-form crystal structure such as chitin derived from cuttlebones and the like. The test of crabs and shrimps is often regarded as industrial waste and preferable as a starting material since it is easily available and effectively used. On the other hand, it requires a protein removing step and a decalcification step to remove protein, minerals and the like contained as impurities. In the present invention, therefore, purified chitin that underwent a matrix removal treatment is preferably used. Purified chitin is commercially available. The starting material for the chitin nanofiber to be used in the present invention may be a chitin having any of the α type and β type crystal structures, but an α type chitin is preferable.

By pulverizing the aforementioned polysaccharides, a nanofiber constituted of the polysaccharides can be obtained. While the pulverization method is not limited, a method affording a strong shear force such as a medium stirring mill, for example, a high-pressure homogenizer, a grinder (stone mill), a bead mill and the like is preferable for subdivision to the below-mentioned fiber diameter and fiber length meeting the object of the present invention.

Of these, subdivision by a high-pressure homogenizer is preferable, and, for example, subdivision (pulverization) by the wet grinding method disclosed in JP-A-2005-270891 or JP-B-5232976 is desirable. Specifically, the starting material is pulverized by spraying a dispersion of a starting material from a pair of nozzles at a high-pressure and bombarding each other, and, for example, Star Burst system (high-pressure pulverization device manufactured by Sugino Machine Limited) or NanoVater (high-pressure pulverization device of yoshida kikai co., ltd.) is used therefor.

In the subdivision (pulverization) of a starting material by the aforementioned high-pressure homogenizer, the degree of subdivision and homogenization depends on the pressure in pumping into an ultrahigh-pressure chamber in a high-pressure homogenizer, and the number (treatment number) of passage through the ultrahigh-pressure chamber, and the concentration of the starting material in the aqueous dispersion. The pumping pressure (treatment pressure) is not particularly limited and it is generally 50 - 250 MPa, preferably 100 - 200 MPa.

While the concentration of the starting material in a aqueous dispersion during the subdividing treatment is not particularly limited, it is generally 0.1 mass % - 30 mass %, preferably 1 mass % - 10 mass %. While the treatment number of the subdivision (pulverization) is not particularly limited, it varies depending on the concentration of the starting material in the aforementioned aqueous dispersion. When the concentration of the starting material is 0.1 - 1 mass %, the treatment number of 10 - 100 is sufficient for pulverization, but 1 - 10 mass % sometimes requires about 10 - 1000 times of treatment.

The viscosity of the aqueous dispersion during the aforementioned subdivision treatment is not particularly limited. For example, in the case of α chitin, the viscosity of the aqueous dispersion is within the range of 1 - 100 mPa·S, preferably 1 - 85 mPa·S (by tuning fork vibration type viscometer (SV-1A, A&D Company Ltd.) under 25°C conditions). In the case of chitosan, the viscosity of the water dispersion is within the range of 0.7 - 30 mPa·S, preferably 0.7 - 10 mPa·S (by tuning fork vibration type viscometer (SV-1A, A&D Company Ltd.) under 25°C conditions).

The preparation method of a nanofiber is described in WO 2015/111686 A1 and the like.

In the production method of the present invention, the nanofiber composed of chitin carries vitronectin in the first step. In the present specification, that the nanofiber carries an extracellular matrix means a state in which the nanofiber and the extracellular matrix are attached or adsorbed without a chemical covalent bond. An extracellular matrix can be carried by nanofibers by intermolecular force, electrostatic interaction, hydrogen bond, hydrophobic interaction, or the like, though it is not limited to these. In other words, the state in which nanofibers carry an extracellular matrix is a state in which the nanofibers and the extracellular matrix remain in contact with each other without a chemical covalent bond, or the nanofibers and the extracellular matrix form a complex without a chemical covalent bond.

In the first step of the production method of the present invention, the extracellular matrix carried on the nanofibers is vitronectin. The selection of the extracellular matrix depends on the type of cells to be proliferated, and can be appropriately selected by those of ordinary skill in the art. For example, in the case of mesenchymal stem cells, vitronectin is preferable as the extracellular matrix. When the vitronectin is human-derived vitronectin, the amino acid sequence thereof (hereinafter referred to as a.a. sequence) preferably consists of 20-398 (SEQ ID NO: 1) or 62-478 (SEQ ID NO: 2). When using non-human-derived vitronectin, a region corresponding to a fragment of human-derived vitronectin can be used.

In the production method of the present invention, the amount of extracellular matrix carried by the nanofiber is generally 0.001 - 50 mg, preferably 0.01 - 10 mg, more preferably 0.1 - 10 mg, further preferably 0.3 - 10 mg, further more preferably, 1 - 10 mg, particularly preferably 2 - 10 mg, per 1 g of nanofibers, though not limited to these.

In the production method of the present invention, the nanofibers carrying vitronectin are prepared by mixing a dispersion in which the nanofibers are dispersed in an aqueous solvent and an aqueous solution of vitronectin, and allowing the mixture to stand for a given period of time as necessary. Examples of the aqueous solvent for dispersing the nanofibers include, but are not limited to, water, dimethyl sulfoxide (DMSO) and the like. As the aqueous solvent, water is preferred. The aqueous solvent may contain appropriate buffering agents and salts. In order to uniformly contact vitronectin with the nanofibers, it is preferable to sufficiently mix them by a pipetting operation or the like. As the time of standing, a mixture of the nanofiber dispersion and vitronectin aqueous solution may be left standing generally for 30 min or more, preferably 1 hr or more, more preferably 3 hr or more, further preferably 6 hr or more, further more preferably 9 hr or more, particularly preferably 12 hr or more. While there is no particular upper limit on the standing time, for example, an upper limit of 48 hr or less (e.g., 36 hr or less, 24 hr or less, 16 hr or less, etc.) may be set. The temperature during standing is not particularly limited, and is generally 1 - 30°C, preferably 1 - 15°C, more preferably 2 - 10°C, particularly preferably 2 - 5°C (e.g., 4°C).

The mixing ratio of the nanofiber composed of chitin and vitronectin varies depending on the kind of these substances to be used, and is, but not limited to, for example, 100:0.1 - 1, preferably, 100:0.4 - 0.6, in terms of the solid content weight.

The amount of vitronectin carried on the nanofibers composed of chitin can be measured by, for example, Micro BCA method, enzyme immunoassay (ELISA method) and the like, but is not limited thereto.

In a preferred embodiment, the nanofibers composed of chitin are uniformly dispersed in a liquid medium, and the adherent cells attached to the nanofibers are suspended in the liquid medium.

In the second step of the production method of the present invention, the medium to which the nanofibers carrying vitronectin are added can be appropriately selected according to the kind and the like of the adherent cells to be used. For example, when used for the purpose of culture of mammalian adherent cells, a medium generally used for culturing mammalian cells can be used as a medium. Examples of the medium for mammalian cells include Dulbecco's Modified Eagle's medium (DMEM), hamF12 medium (Ham's Nutrient Mixture F12), DMEM/F12 medium, McCoy's 5A Medium, Eagle MEM medium (Eagle's Minimum Essential medium; EMEM), αMEM medium (alpha Modified Eagle's Minimum Essential medium; αMEM), MEM medium (Minimum Essential medium), RPMI1640 medium, Iscove's Modified Dulbecco's medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpan SFEM (manufactured by STEMCELL Technologies), StemlineII (manufactured by Sigma Aldrich), QBSF-60 (manufactured by Quality Biological), StemPro hESC SFM (manufactured by Invitrogen), mTeSR1 or 2 medium (manufactured by STEMCELL Technologies), Sf-900II (manufactured by Invitrogen), Opti-Pro (manufactured by Invitrogen), and the like.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture of mammalian cells, those of ordinary skill in the art can also add, according to the object, one or more kinds of other chemical components and biogenic substances in combination. Examples of the components to be added to a medium for mammalian cells include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various proliferation factors, various extracellular matrices, various cell adhesion molecules and the like. Examples of the cytokine to be added to a medium include, but are not limited to, interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-18 (IL-18), interleukin-21 (IL-21), interferon-α (IFN-α), interferon-β (IFN-β), interferon-γ (IFN-γ), granulocyte colony stimulating factor (G-CSF), monocyte colony stimulating factor (M-CSF), granulocyte-macrophage colony stimulating factor (GM-CSF), stem cell factor (SCF), flk2/flt3 ligand (FL), leukemia cell inhibitory factor (LIF), oncostatin M (OM), erythropoietin (EPO), thrombopoietin (TPO) and the like.

Examples of the hormone to be added to a medium include, but are not limited to, melatonin, serotonin, thyroxine, triiodothyronine, epinephrine, norepinephrine, dopamine, anti-Mullerian hormone, adiponectin, adrenocorticotropic hormone, angiotensinogen and angiotensin, antidiuretic hormone, atrial natriuretic peptide, calcitonin, cholecystokinin, corticotropin release hormone, erythropoietin, follicle stimulating hormone, gastrin, ghrelin, glucagon, gonadotropin release hormone, growth hormone release hormone, human chorionic gonadotropin, human placental lactogen, growth hormone, inhibin, insulin, insulin-like growth factor, leptin, luteinizing hormone, melanocyte stimulating hormone, oxytocin, parathyroid hormone, prolactin, secretin, somatostatin, thrombopoietin, thyroid-stimulating hormone, thyrotropin releasing hormone, cortisol, aldosterone, testosterone, dehydroepiandrosterone, androstenedione, dihydrotestosterone, estradiol, estrone, estriol, progesterone, calcitriol, calcidiol, prostaglandin, leukotriene, prostacyclin, thromboxane, prolactin releasing hormone, lipotropin, brain natriuretic peptide, neuropeptide Y, histamine, endothelin, pancreas polypeptide, rennin and enkephalin.

Examples of the growth factor to be added to a medium include, but are not limited to, transforming growth factor-α (TGF-α), transforming growth factor-β (TGF-β), macrophage inflammatory protein-1α (MIP-1α), epithelial cell growth factor (EGF), fibroblast growth factor-1, 2, 3, 4, 5, 6, 7, 8 or 9 (FGF-1, 2, 3, 4, 5, 6, 7, 8, 9), nerve cell growth factor (NGF) hepatocyte growth factor (HGF), leukemia inhibitory factor (LIF), protease nexin I, protease nexin II, platelet-derived growth factor (PDGF), choline vasoactive differentiation factor (CDF), chemokine, Notch ligand (Delta1 and the like), Wnt protein, angiopoietin-like protein 2, 3, 5 or 7 (Angpt2, 3, 5, 7), insulin like growth factor (IGF), insulin-like growth factor binding protein (IGFBP), Pleiotrophin and the like.

In addition, these cytokines and growth factors having amino acid sequences artificially altered by gene recombinant techniques can also be added. Examples thereof include IL-6/soluble IL-6 receptor complex, Hyper IL-6 (fusion protein of IL-6 and soluble IL-6 receptor) and the like.

Examples of the antibiotic to be added to a medium include Sulfonamides and preparations, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, Piromidic acid, ciprofloxacin, cinoxacin, norfloxacin, perfloxacin, Rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, adinoshirin and sulbactam formaldehyde hudrate ester, tazobactam, aztreonam, sulfazethin, isosulfazethin, nocardicin, phenylacetamidophosphonic acid methyl, Chlortetracycline, oxytetracycline, tetracycline, demeclocycline, doxycycline, methacycline, and minocycline.

While the mixing ratio is not particularly limited, nanofiber dispersion:liquid medium (aqueous solution as medium) (volume ratio) is generally 1:99 - 99:1, preferably 10:90 - 90:10, more preferably, 20:80 - 80:20.

Suspending of cells in the present specification refers to a state where cells do not adhere to a culture container (non-adhesive), and whether the cell forms sediment is not questioned. Furthermore, in the present specification, when the cells are cultured, the state where the cells and/or tissues are dispersed and suspended in the liquid medium composition in the absence of a pressure on or vibration of the liquid medium composition from the outside or shaking, rotating operation and the like in the composition is referred to as "suspension standing", and cultivation of the cells and/or tissues in such condition is referred to as "suspension standing culture". In the "suspension standing", the period of suspending includes at least 5 min, preferably, not less than 1 hr, not less than 24 hr, not less than 48 hr, not less than 6 days, not less than 21 days, though the period is not limited thereto as long as the suspended state is maintained.

In a preferable embodiment, the medium composition produced by the production method of the present invention permits suspension standing of cells at least on one point in the temperature range (e.g., 0 - 40°C) capable of culturing cells. The medium composition permits suspension standing of cells at least on one point in the temperature range of preferably 25 - 37°C, most preferably 37°C.

Suspension culture of adherent cells can be performed by culturing adherent cells while being attached to nanofibers carrying extracellular matrix. Nanofibers carrying extracellular matrix show an effect of suspending the cells attached to the nanofibers in the medium (preferably effect of suspension standing) and cell proliferation promotion. Since nanofibers carrying extracellular matrix are dispersed without dissolving or attaching to a culture container in the liquid medium, when adherent cells are cultured in the liquid medium composition, the adherent cells attach to the nanofibers and are suspended in the medium composition. By the suspending effect, a more increased amount of the number of cells per a given volume can be cultivated as compared with monolayer culture. In conventional suspension culture accompanying rotation or shaking operation, the proliferation rate and recovery rate of the cells may become low, or the function of the cell may be impaired since a shear force acts on the cells. Using the medium composition produced by the production method of the present invention, the cells can be cultured in a dispersion state without requiring an operation such as shaking and the like. Thus, easy suspension culture of a large amount of the object adherent cells without loss of the cell function can be expected. In addition, when cells are suspension cultured in a conventional medium containing a gel substrate, observation and recovery of the cells are sometimes difficult, and the function thereof is sometimes impaired during recovery. However, using the medium composition produced by the production method of the present invention, the cells under suspension culture are expected to be observed and recovered without impairing the function thereof. In addition, a conventional medium containing a gel substrate sometimes shows high viscosity that makes it difficult to exchange the medium. However, since the medium composition produced by the production method of the present invention has low viscosity, it is expected to be exchanged easily with a pipette, pump and the like.

When suspension culture of adherent cells is performed using nanofibers carrying extracellular matrix, adherent cells prepared separately are added to the medium composition containing the nanofibers and mixed uniformly. In this case, the mixing method is not particularly limited and, for example, manual mixing using pipetting and the like, mixing using instrument such as stirrer, vortex mixer, microplate mixer, shaking machine and the like can be mentioned. After mixing, the obtained cell suspension may be cultured while being stood still, or cultured with rotation, shaking or stirring as necessary. The rotating speed and frequency can be appropriately set according to the object of those of ordinary skill in the art. For example, adherent cells are recovered from the passage culture, dispersed to a single cell or close thereto using an appropriate cell dissociation solution, the dispersed adherent cells are suspended in the medium composition, and this is subjected to suspension culture (preferably, suspension standing culture).

The temperature when cells are cultivated is generally 25 to 39°C (e.g., 37°C), preferably 33 to 39°C, for animal cells. The CO₂ concentration is generally 4 to 10% by volume in the culture atmosphere, and 4 to 6% volume is preferable. The culture period may be set as appropriately according to the object of the culture.

When adherent cells are cultivated in the medium composition produced by the production method of the present invention, culture vessels generally used for cell culture such as schale, flask, plastic bag, Teflon (registered trade mark) bag, dish, schale, dish for tissue culture, multidish, microplate, microwell plate, multiplate, multiwell plate, chamber slide, tube, tray, culture bag, roller bottle and the like can be used for cultivation. These culture containers are desirably low cell -adhesive so that the adherent cells attached to a nanofiber will not adhere to the culture container. As a low cell -adhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used.

When the medium needs to be exchanged, the cells are separated by centrifugation or filtration treatment, and a fresh medium or the medium composition produced by the production method of the present invention can be added of the cells. Alternatively, the cells are appropriately concentrated by centrifugation or filtration treatment, and a fresh medium or the medium composition of the present invention can be added to the concentrated liquid. For example, unlimitatively, the gravitational acceleration (G) of centrifugation is 100G to 400G, and the size of the pore of the filter used for the filtration treatment is 10 µm to 100 µm.

The adherent cells can also be cultured by automatically conducting cell seeding, medium exchange, cell image obtainment, and recovery of cultured cells, under a mechanical control and under a closed environment while controlling pH, temperature, oxygen concentration and the like and using a bioreactor and an automatic incubator capable of high-density culture.

Since adherent cells are efficiently proliferated when they are subjected to suspension culture while being attached to nanofibers carrying extracellular matrix, the suspension culture is superior as a maintenance or proliferation method of the adherent cells. When adherent cells are subjected to suspension culture while being attached to nanofibers carrying extracellular matrix, they are dispersed while spreading three-dimensionally, without adhering to a culture container or without being locally present only on the bottom surface of the culture container, whereby the proliferation is promoted. As a result, the proliferated cells are connected like cluster of grapes on the nanofiber. This proliferation-promoting effect only requires presence of nanofibers at a concentration sufficient for suspending adherent cells (i.e., avoiding adhesion of adherent cells to culture container) in the medium composition, and capability of suspension standing (i.e., cells being uniformly dispersed and in a suspended state in liquid medium composition, without the presence of pressure, trembling, shaking, rotating operation and the like from the outside) is not essential.

When adherent cells are subjected to suspension culture while being attached to nanofibers carrying extracellular matrix to proliferate the adherent cells, a medium permitting proliferation of the adherent cells while maintaining the character thereof is used as the medium to be used for the suspension culture. Those of ordinary skill in the art can appropriately select the medium according to the kind of the adherent cells.

In another embodiment of the production method of the present invention, chitosan nanofibers may be further blended in addition to the nanofibers carrying vitronectin. By suspension culturing adherent cells using a medium composition containing nanofibers carrying vitronectin and chitosan nanofibers, the proliferation of the cells to be cultured can be promoted and the quality thereof can be maintained. For example, when mammalian stem cells (e.g., mesenchymal stem cell) are suspension cultured, they can be proliferated while maintaining the differentiation potency and homing/chemotactic activity thereof. Therefore, in this embodiment, high-quality stem cells can be prepared in large amount. Whether the stem cell maintains properties such as differentiation potency, chemotactic activity and the like can be determined by a method known per se. Briefly, it can be easily judged by determining, at mRNA level and/or protein level, the expression levels of cell markers relating to undifferentiated state in stem cells (e.g., OCT4 gene, SOX2 gene, NANOG gene, etc.) and cell markers relating to chemotacticity (e.g., CXCR4 gene, etc.).

In this embodiment, to prepare a medium containing a chitin nanofiber carrying vitronectin and a chitosan nanofiber at a desired ratio (weight), they are blended at nanofiber carrying vitronectin:chitosan nanofiber = 1:0.5 - 20 (preferably nanofiber carrying vitronectin:chitosan nanofiber = 1:0.5 - 10, more preferably nanofiber carrying vitronectin:chitosan nanofiber = 1:0.7 - 9, further preferably nanofiber carrying vitronectin:chitosan nanofiber = 1:1 - 8, further more preferably nanofiber carrying vitronectin:chitosan nanofiber = 1:2 - 7, particularly preferably nanofiber carrying vitronectin:chitosan nanofiber = 1:3 - 6). The obtained mixture of nanofibers carrying vitronectin /chitosan nanofibers can be blended in a liquid medium such that the concentration of the total nanofibers (nanofiber carrying vitronectin and chitosan nanofiber) contained in the medium composition is generally 0.0001 - 0.2%(w/v), preferably 0.0005 - 0.1%(w/v), further preferably 0.001 - 0.05%(w/v), particularly preferably 0.006 - 0.05%(w/v). Alternatively, a desired medium may be prepared by separately adding the necessary amounts ofchitin nanofiber carrying vitronectin, and chitosan nanofiber to a liquid medium and stirring well.

In one embodiment, the concentration of the chitin nanofiber carrying vitronectin and the chitosan nanofiber in a medium composition produced by the production method of the present invention satisfies the following conditions:
(1) the concentration of the total nanofibers (nanofiber carrying vitronectin and chitosan nanofiber) contained in the medium composition is 0.0001 - 0.2%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(2) the concentration of the total nanofibers (nanofiber carrying vitronectin and chitosan nanofiber) contained in the medium composition is 0.0005 - 0.1%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(3) the concentration of the total nanofibers (nanofiber carrying vitronectin and chitosan nanofiber) contained in the medium composition is 0.001 - 0.05%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6); or
(4) the concentration of the total nanofibers (nanofiber carrying vitronectin and chitosan nanofiber) contained in the medium composition is 0.006 - 0.05(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6).

In another embodiment, the obtained mixture of nanofiber carrying vitronectin /chitosan nanofiber can be blended in a liquid medium such that the concentration of the total nanofibers (nanofiber carrying vitronectin and chitosan nanofiber) contained in the medium composition is generally 0.0001 - 1.0%(w/v), preferably 0.001 - 0.5%(w/v), further preferably 0.005 - 0.3%(w/v), particularly preferably 0.01 - 0.1%(w/v).

In another embodiment, the concentration of the chitin nanofiber carryingvitronectin and the chitosan nanofiber in a medium composition produced by the production method of the present invention satisfy the following conditions:
(5) the concentration of the total nanofibers (nanofiber carrying vitronectin and chitosan nanofiber) contained in the medium composition is 0.0001 - 1.0%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(6) the concentration of the total nanofibers (nanofiber carrying vitronectin and chitosan nanofiber) contained in the medium composition is 0.001 - 0.5%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(7) the concentration of the total nanofibers (nanofiber carrying vitronectin and chitosan nanofiber) contained in the medium composition is 0.005 - 0.3%(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6); or
(8) the concentration of the total nanofibers (nanofiber carrying vitronectin and chitosan nanofiber) contained in the medium composition is 0.01 - 0.1(w/v), and the weight ratio of nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6).

When adherent cells are subjected to suspension culture while being attached to nanofibers carrying extracellular matrix, the adherent cells can be passaged by simply adding a fresh medium or the medium composition of the present invention to the suspension culture, or just adding a culture after culture entirely or partly to a fresh medium or the medium composition of the present invention, without a detaching operation of the cells from a culture container. Therefore, using the passage culture method of the present invention, adherent cells can be passaged without a detaching operation of the cells from a culture container. Using this passage culture method, moreover, the culture scale of adherent cells can be expanded without a detaching operation of the cells from a culture container. As a detaching operation of the cells from a culture container, a treatment with a chelating agent (e.g., EDTA) and/or protease (e.g., trypsin, collagenase) can be mentioned. The above-mentioned passage culture method is advantageous for passage culture of adherent cells highly sensitive to a detaching operation of the cells from a culture container (e.g., adherent cell with viability decreased by detaching operation, adherent cell with character susceptible to change by detaching operation). Examples of the adherent cells highly sensitive to a detaching operation of the cells from a culture container include, but are not limited to, stem cell (e.g., mesenchymal stem cell), progenitor cell (e.g., pre-adipocyte), primary cultured cells and the like.

For example, a degrading enzyme of chitin is added to a suspension of adherent cells attached to chitin nanofibers and the mixture is incubated for a time sufficient for the detachment of the adherent cells. The temperature of incubation by the degrading enzyme of chitin is generally 20°C - 37°C. The incubation time varies depending on the kind of the enzyme and the like and is generally 5 - 60 min.

When the chitin nanofiber is degraded and the adherent cells are detached from the nanofiber, the detached adherent cells can be recovered by subjecting the suspension to centrifugation.

Since the damage on the thus-recovered adherent cells is minimized, the cells can be preferably used for functional analysis, transplantation and the like.

### 2. Composition for addition to medium

The present invention also provides a composition for addition to a medium, which contains chitin nanofibers carrying vitronectin and chitosan nanofibers, wherein carrying is a state in which the nanofiber and vitronectin are attached or adsorbed without a chemical covalent bond (hereinafter sometimes referred to as "the composition of the present invention").

The composition of the present invention characteristically contains chitin nanofibers carrying vitronectin and chitosan nanofibers. The chitin nanofiber, chitosan nanofiber, vitronectin, and the like in the composition of the present invention are the same as those described in the production method of the present invention.

The following method is exemplified as one embodiment of the method for preparing the composition of the present invention. First, an aqueous solution of vitronectin is mixed with a dispersion composed of chitin nanofibers and an aqueous solvent, and the mixture is stirred and then allowed to stand (conditions such as time, temperature, and the like during standing are the same as those described in "the production method of the present invention"). Stirring is not particularly limited, and may be performed by a pipetting operation or the like. As a result, a dispersion of chitin nanofibers carrying vitronectin is prepared. Then, the composition of the present invention can be prepared by blending a dispersion composed of chitosan nanofibers and an aqueous solvent with a dispersion of chitin nanofibers carrying vitronectin and stirring the mixture.

The amount of vitronectin carried by the chitin nanofiber contained in the composition of the present invention is not particularly limited as long as the desired effect can be obtained. The amount of vitronectin is generally 0.001 - 50 mg, preferably 0.01 - 10 mg, more preferably 0.1 - 10 mg, further preferably 0.3 - 10 mg, further more preferably, 1 - 10 mg, particularly preferably 2 - 10 mg, per 1 g of chitin nanofiber, though not limited to these.

In the composition of the present invention, the quantitative ratio (weight) of the chitin nanofiber carrying vitronectin and the chitosan nanofiber is not particularly limited as long as the desired effect can be obtained. It is generally chitin nanofiber carrying vitronectin:chitosan nanofiber = 1:0.5 - 20 (preferably chitin nanofiber carrying vitronectin:chitosan nanofiber = 1:0.5 - 10, more preferably chitin nanofiber carrying vitronectin:chitosan nanofiber = 1:0.7 - 9, further preferably chitin nanofiber carrying vitronectin:chitosan nanofiber = 1:1 - 8, further more preferably chitin nanofiber carrying vitronectin:chitosan nanofiber = 1:2 - 7, particularly preferably chitin nanofiber carrying vitronectin:chitosan nanofiber = 1:3 - 6).

The composition of the present invention is blended with a culture medium of adherent cells. The amount of the composition of the present invention to be added to a medium is not particularly limited as long as the desired effect can be obtained. It can be added such that, for example, the concentration of the total nanofiber (chitin nanofiber carrying vitronectinand chitosan nanofiber) contained in the medium is generally 0.0001 - 0.2%(w/v), preferably 0.0005 - 0.1%(w/v), further preferably 0.001 - 0.05%(w/v), particularly preferably 0.006 - 0.05%(w/v).

In another embodiment, the composition of the present invention can be added to the medium in an amount that makes the concentration of the total nanofibers (chitin nanofiber carrying vitronectinand chitosan nanofiber) contained in the medium generally 0.0001 - 1.0%(w/v), preferably 0.001 - 0.5%(w/v), further preferably 0.005 - 0.3%(w/v), particularly preferably 0.01 - 0.1%(w/v).

### 3. Medium composition

The present invention also provides a medium composition for suspension culture of an adherent cell, containing the composition of the present invention (hereinafter sometimes to be referred to as "the medium composition of the present invention").

The medium composition of the present invention is prepared by mixing the composition of the present invention with a medium for cell culture. The medium for cell culture can be appropriately determined according to the kind of adherent cells to be cultured. Examples of the medium used for preparing the medium composition of the present invention include the media exemplified in "the production method of the present invention".

In one embodiment, the chitin nanofiber and chitosan nanofiber carrying vitronectin in the medium composition of the present invention satisfies the following conditions:
(1) the concentration of the total nanofibers (chitin nanofiber carrying vitronectinand chitosan nanofiber) contained in the medium composition is 0.0001 - 0.2%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(2) the concentration of the total nanofibers (chitin nanofiber carrying vitronectinand chitosan nanofiber) contained in the medium composition is 0.0005 - 0.1%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(3) the concentration of the total nanofibers (chitin nanofiber carrying vitronectinand chitosan nanofiber) contained in the medium composition is 0.001 - 0.05%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6); or
(4) the concentration of the total nanofibers (chitin nanofiber carrying vitronectinand chitosan nanofiber) contained in the medium composition is 0.006 - 0.05(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6).

In another embodiment, the chitin nanofiber and chitosan nanofiber carrying vitronectin in the medium composition of the present invention satisfies the following conditions:
(5) the concentration of the total nanofibers (chitin nanofiber carrying vitronectinand chitosan nanofiber) contained in the medium composition is 0.0001 - 1.0%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(6) the concentration of the total nanofibers (chitin nanofiber carrying vitronectinand chitosan nanofiber) contained in the medium composition is 0.001 - 0.5%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6);
(7) the concentration of the total nanofibers (chitin nanofiber carrying vitronectinand chitosan nanofiber) contained in the medium composition is 0.005 - 0.3%(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6); or
(8) the concentration of the total nanofibers (chitin nanofiber carrying vitronectinand chitosan nanofiber) contained in the medium composition is 0.01 - 0.1(w/v), and the weight ratio of chitin nanofiber carrying vitronectin:chitosan nanofiber contained in the medium composition is 1:0.5 - 20 (preferably, 1:0.5 - 10, 1:0.7 - 9, 1:1 - 8, 1:2 - 7, or 1:3 - 6).

The present invention is explained more specifically in the following Examples. However, the present invention is not limited in any way by the Examples.

### [Example]

### [Preparation Example 1] (Reference example)

### (Preparation of aqueous dispersion containing chitin nanofibers)

A 2 mass % chitin nanofiber aqueous dispersion prepared according to the description of the above-mentioned patent document 1 (WO 2015/111686) was subjected to an autoclave sterilization treatment at 121°C for 20 min. Thereafter, the aqueous dispersion was mixed and suspended in aseptically distilled water (OTSUKA DISTILLED WATER, manufactured by Otsuka Pharmaceutical Factory, Inc.) at 1%(w/v) to produce an aqueous dispersion containing aseptic chitin nanofibers.

### [Preparation Example 2] (Reference example)

### (Preparation of aqueous dispersion containing chitosan nanofibers)

A 2 mass % chitosan nanofiber aqueous dispersion prepared according to the description of the above-mentioned patent document 1 (WO 2015/111686) was subjected to an autoclave sterilization treatment at 121°C for 20 min. Thereafter, the aqueous dispersion was mixed and suspended in aseptically distilled water (OTSUKA DISTILLED WATER, manufactured by Otsuka Pharmaceutical Factory, Inc.) at 1%(w/v) to produce an aqueous dispersion containing aseptic chitosan nanofibers.

### [Preparation Example 3] (Reference example)

### (Preparation of aqueous dispersion containing chitin nanofibers and chitosan nanofibers)

To the chitin nanofiber aqueous dispersion (2 mL) produced in Preparation Example 1 was added the chitosan nanofiber aqueous dispersion (8 mL) produced in Preparation Example 2, and they were mixed by pipetting to produce an aqueous dispersion (10 mL) containing chitin nanofibers and chitosan nanofibers.

### [Preparation Example 4]

### (Preparation of aqueous dispersion containing vitronectin-carrying chitin nanofibers)

To the 1%(w/v) chitin nanofiber aqueous dispersion produced in Preparation Example 1 was added an aqueous solution containing 500 µg/mL vitronectin (Gibco Vitronectin (VTN-N) Recombinant Human Protein, Truncated, manufactured by Thermo Fisher Scientific), they were mixed by pipetting and stood at 4°C overnight to produce aqueous dispersions containing vitronectin-carrying chitin nanofibers with different amounts of vitronectin. The produced aqueous dispersions containing vitronectin-carrying chitin nanofibers are shown in Table 1. Analysis Example 1 noted below confirmed that vitronectin was carried on the chitin nanofibers.

**[Table 1]**

| No. | 1%(w/v) chitin nanofiber aqueous dispersion (mL) | 500 µg/mL vitronectin aqueous solution (mL) |
|---|---|---|
| DHd509 | 5 | 0.1 |
| DHd510 | 5 | 0.2 |
| DHd511 | 5 | 0.5 |

### [Preparation Example 5]

### (Preparation of aqueous dispersion containing vitronectin-carrying chitin nanofibers and chitosan nanofibers)

To the vitronectin-carrying chitin nanofiber aqueous dispersion (2 mL) produced in Preparation Example 4 was added the chitosan nanofiber aqueous dispersion (8 mL) produced in Preparation Example 2, and they were mixed by pipetting to produce an aqueous dispersion (10 mL) containing vitronectin-carrying chitin nanofibers and chitosan nanofibers. The produced aqueous dispersions containing vitronectin-carrying chitin nanofibers and chitosan nanofibers are shown in Table 2.

**[Table 2]**

| No. | 1%(w/v) vitronectin-carrying chitin nanofiber aqueous dispersion (mL) | 1%(w/v) chitosan nanofiber aqueous dispersion (mL) |
|---|---|---|
| DHd513 | 2 (DHd509) | 8 |
| DHd514 | 2 (DHd510) | 8 |
| DHd515 | 2 (DHd511) | 8 |

### [Analysis Example 1]

### (Staining vitronectin in compositions)

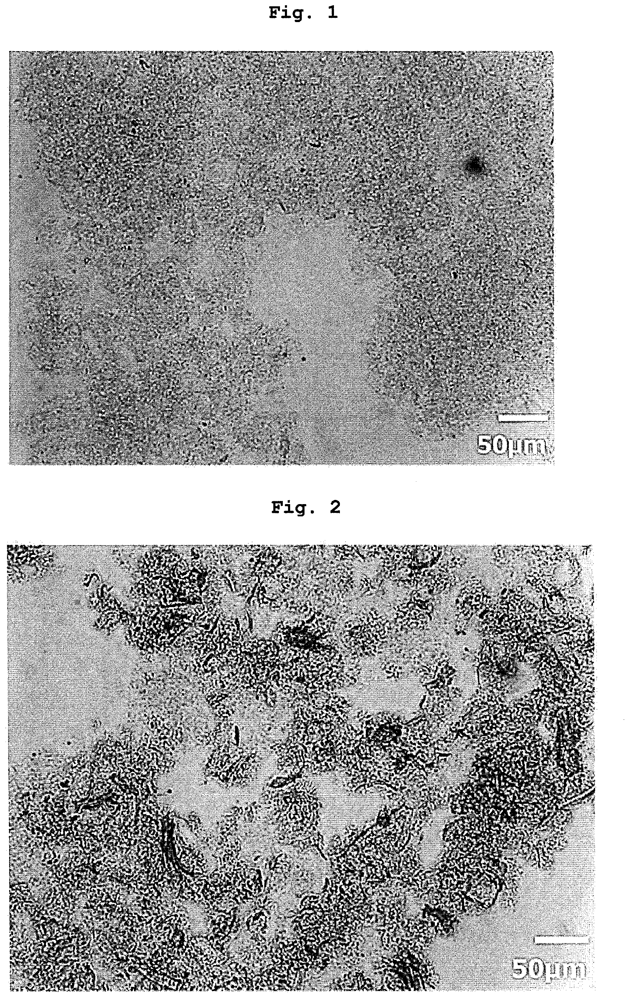

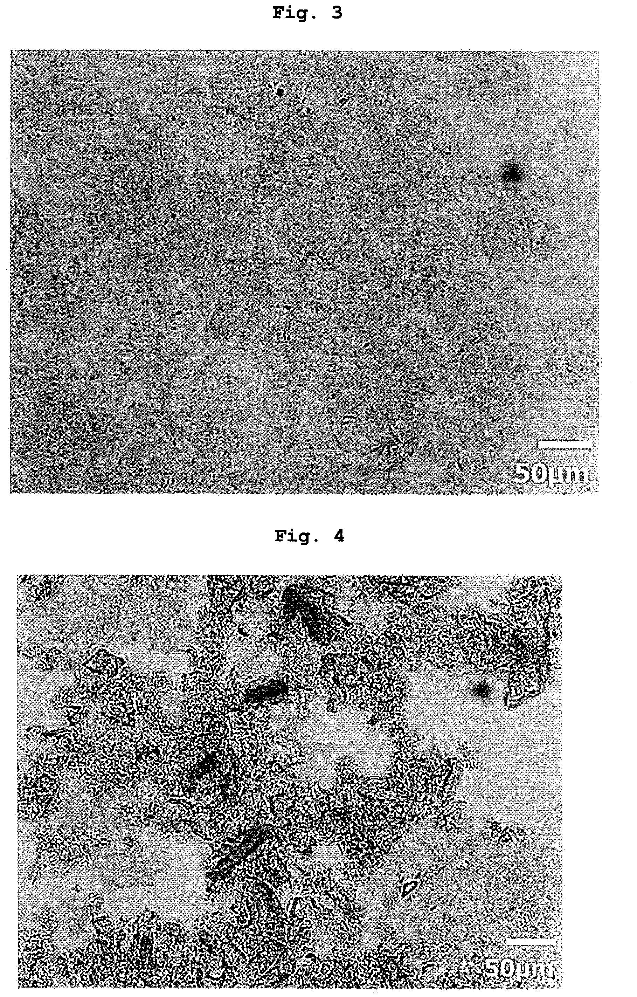

To each of the chitin nanofiber aqueous dispersion (100 µL) produced in Preparation Example 1, the chitin nanofiber/chitosan nanofiber aqueous dispersion (100 µL) produced in Preparation Example 3, DHd511 (100 µL) produced in Preparation Example 4, and DHd515 (100 µL) produced in Preparation Example 5 was added a protein sensitive staining solution (TaKaRa CBB Protein Safe Stain, manufactured by Takara Bio Inc.) (40 µL), and the mixtures were stood for 10 min. These were dropped onto a glass plate by 20 µL each, covered with a cover glass, and observed with a phase contrast microscope. The observation image of the chitin nanofiber aqueous dispersion is shown in Fig. 1, the observation image of the chitin nanofiber/chitosan nanofiber aqueous dispersion is shown in Fig. 2, the observation image of the DHd511 is shown in Fig. 3, and the observation image of the DHd515 is shown in Fig. 4. No stained part was observed in Fig. 1 and Fig. 2, whereas staining was observed in the chitin nanofiber part in Fig. 3 and Fig. 4. From this, it was confirmed that vitronectin was physically adsorbed and carried on the surface of the chitin nanofiber by the method of Preparation Example 4. This is considered to be attributable to the physical adsorption of vitronectin onto the chitin nanofibers (rather than chemical bonds). It was also shown from Fig. 4 that vitronectin was still carrieded on the chitin nanofibers even after the step of Preparation Example 5.

### [Analysis Example 2]

### (Calculation of vitronectin amount carried by chitin fibers)

The vitronectin-carrying chitin nanofiber aqueous dispersion produced in Preparation Example 4 contains only vitronectin as a protein. Therefore, the amount of vitronectin carried was calculated by the following method using Micro BCA Protein Assay Kit (manufactured by Thermo Fisher Scientific), which is a kit for total protein quantification.

DHd509, 510, 511 (1 mL each) produced in Preparation Example 4 were respectively dispensed in 1.5 mL microtubes, and centrifuged (12300×g, 5 min). The supernatant (500 µL) was filtered through a 0.45 µm filter vial (manufactured by Thomason), and the filtrate (300 µL) was collected in new 1.5 mL microtubes. With reference to the protocol of the aforementioned kit, Working Reagent (300 µL) was prepared by mixing MA solution (5.0 mL)/MB solution (4.8 mL)/MC solution (0.2 mL) and added to the recovered supernatant, and the mixture was heated at 60°C for 1 hr. After heating, 300 µL each was dispensed into a 96-well assay plate (Corning 3603), and the absorbance at 562 nm was measured with a plate reader (infinite M200PRO, manufactured by Tecan). The calibration curve of vitronectin was prepared by treating 1, 5, 25, 50, 100 µg/mL solutions in the same manner as in the above-mentioned sample and using the absorbance measured at 562 nm. From the amount of vitronectin contained in the supernatant of the aqueous dispersion quantified from the obtained measured values, the amount of vitronectin carried (physically adsorbed) on the surface of chitin was calculated by the following formula. [total amount of added vitronectin] - [amount of vitronectin contained in supernatant of aqueous dispersion] = [amount of vitronectin carried by chitin surface]

The results are shown in Table 3. From Table 3, it was confirmed that the blended vitronectin was carried by chitin nanofibers, and it was found that the amount of vitronectin carried by the chitin nanofibers increased as the addition amount of vitronectin increased.

**[Table 3]**

| Lot. | chitin concentration % (w/v) | VTN-N addition concentration (µg/mL) | VTN-N supernatant concentration (µg/mL) | amount of VTN-N carried (µg/mL) | VTN-N weight/ chitin weight (mg/g) |
|---|---|---|---|---|---|
| DHd509 | 0.98 | 9.8 | 9.72 | 0.08 | 0.01 |
| DHd510 | 0.96 | 19.23 | 14.47 | 4.76 | 0.5 |
| DHd511 | 0.91 | 45.45 | 24.64 | 20.81 | 2.29 |

### [Experimental Example 1]

### (Continuous expansion culture of umbilical cord-derived mesenchymal stem cell in 3D culture using medium composition containing vitronectin-carrying chitin nanofibers and chitosan nanofibers)

DHd513, DHd514, and DHd515 prepared in Preparation Example 5 were respectively added to a mesenchymal stem cell proliferation medium (C-28009, manufactured by Takara Bio Inc.), which is a serum medium, to a final concentration of 0.05% (w/v) to prepare medium compositions. As a control sample, the aqueous dispersion containing 1% (w/v) chitin nanofibers and chitosan nanofibers prepared in Preparation Example 3 was added to a final concentration of 0.05% (w/v) to prepare a medium composition.

Successively, the cultured human umbilical cord-derived mesenchymal stem cells (C-12971, manufactured by Takara Bio Inc.) were respectively suspended in each of the above-mentioned medium compositions at 16667 cells/mL, and seeded in a 24-well flat-bottomed ultra low attachment surface microplate (manufactured by Corning Incorporated, #3473) at 1.2 mL/well. The cells were cultured in a CO₂ incubator (37°C, 5% CO₂) in a static state for 4 days. An ATP reagent (300 µL, CellTiter-Glo^{™} Luminescent Cell Viability Assay, manufactured by Promega) was added to 300 µL of the culture media at the time of seeding (day 0) and day 4 after the seeding, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples.

On day 4 after seeding, the nanofibers to which the cells were adhered were redispersed by pipetting, and the suspension for 2 wells (about 2.4 mL) was collected from a 24-well flat-bottomed ultra-low adhesion surface microplate. Each of the above-mentioned medium composition (7.6 mL) was newly added and mixed by pipetting, and then seeded and cultured in a 6-well flat-bottomed ultra-low-adhesion surface microplate (manufactured by Corning Incorporated, #3471) at total volume/well. On day 7, cell-adhered nanofibers were redispersed by pipetting and about 5 mL of the suspension was recovered from the 6-well flat-bottomed ultra-low-adhesion surface microplate. Thereto was newly added 5 mL each of the above-mentioned medium compositions, mixed by pipetting, and then seeded and cultured in a fresh 6-well flat-bottomed ultra-low-adhesion surface microplate (manufactured by Corning Incorporated, #3471) at total volume/well. The same operation as on day 7 was also performed on day 10, and the culture was continued until day 13. Expansion culture was performed on a 6-well flat-bottomed ultra-low-adhesion surface microplate. An ATP reagent (500 µL, CellTiter-Glo^{™} Luminescent Cell Viability Assay, manufactured by Promega) was added to 500 µL of the cell culture media on days 7, 10, 13, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples. In addition, the final ATP values on days 4, 7, 10, 13 were converted using the expansion ratio at each step.

As a result, when human umbilical cord-derived mesenchymal stem cells were cultured using a medium composition containing vitronectin-carrying chitin nanofibers and chitosan nanofibers, a clear proliferation promoting effect was observed compared with the medium composition containing chitin nanofibers not carrying vitronectin and chitosan nanofibers (control sample). Moreover, the proliferation promoting effect depended on the amount of vitronectin carried. Further, expansion culture was possible by simply adding a medium composition containing fresh chitin nanofibers and chitosan nanofibers, without performing a cell detachment treatment from the substrate with trypsin or the like. The converted RLU values (ATP measurement, luminescence intensity) in each culture are shown in Table 4.

**[Table 4]**

| | day 0 (24 well) | day 4 (24 well) | day 7 (6 well) | day 10 (6 well) | day 13 (6 well) |
|---|---|---|---|---|---|
| on seeding | 1472 | - | - | - | - |
| control sample | (same as above) | 8697 | 20184 | 36471 | 57413 |
| DHd513 | (same as above) | 9053 | 22400 | 48707 | 76209 |
| DHd514 | (same as above) | 9505 | 26863 | 55933 | 123086 |
| DHd515 | (same as above) | 12710 | 47003 | 108722 | 207309 |

### [Experimental Example 2]

### (Continuous expansion culture of human bone marrow-derived mesenchymal stem cell in 3D culture using medium composition containing vitronectin-carrying chitin nanofibers and chitosan nanofibers)

DHd513, DHd514, and DHd515 prepared in Preparation Example 5 were respectively added to a mesenchymal stem cell proliferation medium (C-28009, manufactured by Takara Bio Inc.), which is a serum medium, to a final concentration of 0.05% (w/v) to prepare medium compositions. As a control sample, the aqueous dispersion containing 1% (w/v) chitin nanofibers and chitosan nanofibers prepared in Preparation Example 3 was added to a final concentration of 0.05% (w/v) to prepare a medium composition.

Successively, the cultured human bone marrow-derived mesenchymal stem cells (C-12977, manufactured by Takara Bio Inc.) were respectively suspended in each of the above-mentioned medium compositions at 8333 cells/mL, and seeded in a 24-well flat-bottomed ultra low attachment surface microplate (manufactured by Corning Incorporated, #3473) at 1.2 mL/well. The cells were cultured in a CO₂ incubator (37°C, 5% CO₂) in a static state. On day 4, about 0.6 mL of the medium supernatant in the well was removed, a fresh mesenchymal stem cell proliferation medium (0.6 mL) was added to each well, suspended by pipetting, and culture was continued until day 8 after seeding. An ATP reagent (300 µL, CellTiter-Glo^{™} Luminescent Cell Viability Assay, manufactured by Promega) was added to 300 µL of the culture media at the time of seeding (day 0) and day 8 after the seeding, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples.

On day 8 after seeding, only for DHd515 group, the nanofibers to which the cells were adhered were redispersed by pipetting, and about 0.6 mL was collected from a 24-well flat-bottomed ultra-low adhesion surface microplate. 0.6 mL of a medium composition containing the above-mentioned DHd515 was newly added and mixed by pipetting, and then seeded in a 24-well flat-bottomed ultra-low-adhesion surface microplate (manufactured by Corning Incorporated, #3471) at total volume/well. Culture was continued until day 11 after seeding. For other sample groups, about 0.6 mL of the medium supernatant in the well was removed, a fresh mesenchymal stem cell proliferation medium (0.6 mL) was added to each well, suspended by pipetting, and culture was continued until day 11 after seeding. On day 11, in all sample groups containing DHd515, about 0.6 mL of the medium supernatant in the well was removed, a fresh mesenchymal stem cell proliferation medium (0.6 mL) was added to each well, suspended by pipetting, and culture was continued until day 15 after seeding. On day 15 after seeding, in DHd515 group and DHd514 group, the nanofibers to which the cells were adhered were redispersed by pipetting, and about 0.6 mL of the suspension was recovered from a 24-well flat-bottomed ultra-low adhesion surface microplate. A medium composition (0.6 mL) containing the above-mentioned DHd515 or DHd514 was newly added and mixed by pipetting, and then seeded in a 24-well flat-bottomed ultra-low-adhesion surface microplate (manufactured by Corning Incorporated, #3471) at total volume/well. Culture was continued until day 18 after seeding. In other sample groups, about 0.6 mL of the medium supernatant in the well was removed, a fresh mesenchymal stem cell proliferation medium (0.6 mL) was added to each well, suspended by pipetting, and culture was continued until day 18 after seeding. On day 18, in all sample groups containing DHd515, about 0.6 mL of the medium supernatant in the well was removed, a fresh mesenchymal stem cell proliferation medium (0.6 mL) was added to each well, suspended by pipetting, and culture was continued until day 22 after seeding. On day 22 after seeding, in DHd515 group and DHd514 group, the nanofibers to which the cells were adhered were redispersed by pipetting, and about 0.6 mL of the suspension was recovered from a 24-well flat-bottomed ultra-low adhesion surface microplate. A medium composition (0.6 mL) containing the above-mentioned DHd515 or DHd514 was newly added and mixed by pipetting, and then seeded in a 24-well flat-bottomed ultra-low-adhesion surface microplate (manufactured by Corning Incorporated, #3471) at total volume/well. Culture was continued until day 27 after seeding. In other sample groups, about 0.6 mL of the medium in the well was removed, a fresh mesenchymal stem cell proliferation medium (0.6 mL) was added to each well, suspended by pipetting, and culture was continued until day 27 after seeding.

An ATP reagent (300 µL, CellTiter-Glo^{™} Luminescent Cell Viability Assay, manufactured by Promega) was added to 300 µL of the culture media on days 15, 22, 27 cultured on a 24-well flat-bottomed ultra-low adhesion surface microplate, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples. In addition, the final ATP values on days 15, 22, 27 were converted using the expansion ratio at each step.

As a result, when human bone marrow-derived mesenchymal stem cells were cultured using a medium composition containing vitronectin-carrying chitin nanofibers and chitosan nanofibers, a clear proliferation promoting effect was observed compared with the medium composition containing chitin nanofibers not carrying vitronectin and chitosan nanofibers (control sample). Moreover, the proliferation promoting effect depended on the amount of vitronectin carried. Further, expansion culture was possible by simply adding a medium composition containing fresh chitin nanofibers and chitosan nanofibers, without performing a cell detachment treatment from the substrate with trypsin or the like. The converted RLU values (ATP measurement, luminescence intensity) in each culture are shown in Table 5.

**[Table 5]**

| | day 0 (24 well) | day 8 (24 well) | day 15 (24 well) | day 22 (24 well) | day 27 (24 well) |
|---|---|---|---|---|---|
| on seeding | 514 | - | - | - | - |
| control sample | (same as above) | 1534 | 2724 | 2856 | 2851 |
| DHd513 | (same as above) | 2353 | 3542 | 3192 | 3950 |
| DHd514 | (same as above) | 2921 | 5406 | 13028 | 15453 |
| DHd515 | (same as above) | 5341 | 9135 | 27273 | 54436 |

### [Experimental Example 3]

### (Continuous expansion culture of human adipose-derived mesenchymal stem cell in 3D culture using medium composition containing vitronectin-carrying chitin nanofibers and chitosan nanofibers, and comparison with vitronectin addition effect)

DHd513, DHd514, and DHd515 prepared in Preparation Example 5 were respectively added to a mesenchymal stem cell proliferation medium (C-28009, manufactured by Takara Bio Inc.), which is a serum medium, to a final concentration of 0.05% (w/v) to prepare medium compositions. As a control sample, the aqueous dispersion containing 1% (w/v) chitin nanofibers and chitosan nanofibers prepared in Preparation Example 3 was added to a final concentration of 0.05% (w/v) to prepare a medium composition.

Successively, the cultured human adipose-derived mesenchymal stem cells (C-12977, manufactured by Takara Bio Inc.) were respectively suspended in each of the above-mentioned medium compositions at 8333 cells/mL, and seeded in a 24-well flat-bottomed ultra low attachment surface microplate (manufactured by Corning Incorporated, #3473) at 1.2 mL/well. The cells were cultured in a CO₂ incubator (37°C, 5% CO₂) in a static state. In addition, several hours after seeding using the control sample, an aqueous solution containing 500 µg/mL vitronectin (Gibco Vitronectin (VTN-N) Recombinant Human Protein, Truncated, manufactured by Thermo Fisher Scientific) was added to a final concentration of 0.5 µg/mL or 1.0 µg/mL, and culture was continued. Note that 0.5 µg/mL is an equivalent addition amount to the amount of vitronectin used for carrying in DHd515, and 1.0 µg/mL is the amount of vitronectin which is two times that in DHd515. An ATP reagent (300 µL, CellTiter-Glo^{™} Luminescent Cell Viability Assay, manufactured by Promega) was added to 300 µL of the culture media at the time of seeding (day 0) and day 4 after the seeding, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples.

On day 4 after seeding, in the control sample, about 0.6 mL of the medium supernatant in the well was removed, a fresh mesenchymal stem cell proliferation medium (0.6 mL) was added to each well, suspended by pipetting, and culture was continued until day 8 after seeding. On the other hand, in other samples, the nanofibers to which the cells were adhered were redispersed by pipetting, and about 0.6 mL of the suspension was collected from a 24-well flat-bottomed ultra-low adhesion surface microplate. 0.6 mL of each medium composition was newly added and mixed by pipetting, and then seeded in a 24-well flat-bottomed ultra-low-adhesion surface microplate (manufactured by Corning Incorporated, #3473) at total volume/well. Culture was continued until day 8 after seeding. After day 8 from seeding, at 3 - 4 day intervals, the nanofibers to which the cells were adhered were redispersed by pipetting, and about 0.6 mL of the suspension was collected from the 24-well flat-bottomed ultra-low adhesion surface microplate. 0.6 mL of each medium composition was newly added and mixed by pipetting, and then seeded in a 24-well flat-bottomed ultra-low-adhesion surface microplate (manufactured by Corning Incorporated, #3473) at total volume/well. Culture was continued until day 21 after seeding.

An ATP reagent (300 µL, CellTiter-Glo^{™} Luminescent Cell Viability Assay, manufactured by Promega) was added to 300 µL of the culture media on days 8, 15, 21 cultured on a 24-well flat-bottomed ultra-low adhesion surface microplate, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples. In addition, the final ATP values on days 8, 15, 21 were converted using the expansion ratio at each step.

As a result, when human adipose-derived mesenchymal stem cells were cultured using a medium composition containing vitronectin-carrying chitin nanofibers and chitosan nanofibers, a clear proliferation promoting effect was observed compared with the medium composition containing chitin nanofibers not carrying vitronectin and chitosan nanofibers (control sample). Moreover, the proliferation promoting effect depended on the amount of vitronectin carried. Furthermore, proliferation promoting effect of DHd515 was clearly higher than the effect achieved by directly adding the same or 2-fold amount of vitronectin to a culture medium using the control sample. Further, expansion culture was possible by simply adding a medium composition containing fresh chitin nanofibers and chitosan nanofibers, without performing a cell detachment treatment from the substrate with trypsin or the like. The converted RLU values (ATP measurement, luminescence intensity) in each culture are shown in Table 6.

**[Table 6]**

| | day 0 (24 well) | day 8 (24 well) | day 15 (24 well) | day 21 (24 well) |
|---|---|---|---|---|
| on seeding | 1098 | - | - | - |
| control sample | (same as above) | 4880 | 12942 | 25789 |
| DHd513 | (same as above) | 7613 | 22738 | 46358 |
| DHd514 | (same as above) | 13760 | 49285 | 79574 |
| DHd515 | (same as above) | 27202 | 79337 | 278336 |
| control sample +0.5 µg/mL VTN-N | (same as above) | 8088 | 19571 | 31472 |
| control sample +1.0 µg/mL VTN-N | (same as above) | 11231 | 45190 | 78509 |

### [Experimental Example 4]

### (Study of cell proliferation effect based on difference in amino acid sequence of vitronectin to be carried)

To the 1%(w/v) chitin nanofiber aqueous dispersion (5 mL) produced in Preparation Example 1 was added 0.5 mL of any of 3 types of aqueous solutions of 500 µg/mL vitronectin with different amino acid residues ((A) Gibco Vitronectin (VTN-N) Recombinant Human Protein, Truncated, a.a. sequence 62-478, manufactured by Thermo Fisher Scientific, (B) PluriSTEM-XF Recombinant Vitronectin, a.a. sequence 20-398, manufactured by Sigma-Aldrich, (C) Animal-Free Recombinant Human Vitronectin, HEK293 cell derived, a.a. sequence 20-478 (SEQ ID NO: 3), manufactured by PeproTech), mixed by pipetting, and allowed to stand at 4°C overnight to produce 3 types of vitronectin-carrying chitin nanofiber-containing aqueous dispersions. In the following, the aqueous dispersions containing vitronectin-carrying chitin nanofibers and chitosan nanofibers, which were prepared using the above-mentioned (A), (B), or (C) are respectively denoted as aqueous dispersion A, aqueous dispersion B, or aqueous dispersion C.

Aqueous dispersion A, aqueous dispersion B, or aqueous dispersion C was added to a mesenchymal stem cell proliferation medium (C-28009, manufactured by Takara Bio Inc.), which is a serum medium, to a final concentration of 0.05% (w/v) to prepare a medium composition.

Successively, the cultured human umbilical cord-derived mesenchymal stem cells (C-12971, manufactured by Takara Bio Inc.) were respectively suspended in each of the above-mentioned medium compositions at 15000 cells/mL, and seeded in a 6-well flat-bottomed ultra low attachment surface microplate (manufactured by Corning Incorporated, #3471) at 10 mL/well. The cells were cultured in a CO₂ incubator (37°C, 5% CO₂) in a static state. On day 3, about 5 mL of the medium supernatant in the well was removed, a fresh mesenchymal stem cell proliferation medium (5 mL) was added to each well, suspended by pipetting, thereby exchanging half volume of the medium, and culture was continued until day 7 after seeding. On day 7, the nanofibers to which the cells attached were redispersed by pipetting and 5 mL of the suspension was recovered from the 6-well flat-bottomed ultra-low-adhesion surface microplate. Thereto was newly added 5 mL each of the above-mentioned medium compositions, mixed by pipetting, and then seeded and cultured in a fresh 6-well flat-bottomed ultra-low-adhesion surface microplate (manufactured by Corning Incorporated, #3471) at total volume/well. The same operation as on day 7 was also performed on day 10, and the culture was continued until day 13. An ATP reagent (500 µL, CellTiter-Glo^{™} Luminescent Cell Viability Assay, manufactured by Promega) was added to 500 µL of the cell culture media at the time of seeding (day 0) and days 3, 7, 10, 13 of culture on a 6-well flat-bottomed ultra-low-adhesion surface microplate, and they were suspended and stood for about 10 min at room temperature. The luminescence intensity (RLU value) was measured by FlexStation3 (manufactured by Molecular Devices) and the luminescence value of the medium alone was subtracted to calculate the amount of viable cells as a mean of 2 samples. In addition, the final ATP values on days 3, 7, 10, 13 were converted using the expansion ratio at each step. The converted RLU values (ATP measurement, luminescence intensity) in each culture are shown in Table 7 and Table 8.

As a result, vitronectin in (A) and (B) showed equivalent proliferation effects. On the other hand, vitronectin in (C) showed mild proliferation effect compared with (A).

**[Table 7]**

| | day 0 (6 well) | day 3 (6 well) | day 7 (6 well) | day 10 (6 well) | day 13 (6 well) |
|---|---|---|---|---|---|
| A | 107436 (on seeding) | 383868 | 1487491 | 2035450 | 2440453 |
| B | 107436 (on seeding) | 368956 | 1230836 | 2098070 | 2540106 |

**[Table 8]**

| | day 0 (6 well) | day 3 (6 well) | day 7 (6 well) | day 10 (6 well) | day 13 (6 well) |
|---|---|---|---|---|---|
| A | 116273 (on seeding) | 177803 | 527707 | 1257390 | 2424467 |
| C | 116273 (on seeding) | 182198 | 415918 | 670810 | 894040 |

### [Industrial Applicability]

Adherent cells such as mesenchymal stem cell, pre-adipocyte, and the like can be suspension cultured in a stationary state by using, as a carrier substrate, polysaccharide nanofiber in which extracellular matrix is carried on nanofiber-like polysaccharides that are insoluble in a medium and float in the medium. The cells adhered to the nanofibers show promoted proliferation under suspension culture conditions, and long-term viability.

This application is based on a patent application No. 2019-125536 filed in Japan (filing date: July 4, 2019).

## Claims

1. A method for producing a medium composition for suspension culture of an adherent cell, comprising the following steps:
(i) a step of making vitronectin carried on a nanofiber composed of chitin,
(ii) a step of adding vitronectin-carrying chitin nanofiber obtained in step (i) to a medium,
wherein carried is a state in which the nanofiber and vitronectin are attached or adsorbed without a chemical covalent bond.

2. The method according to claim 1, wherein the chitin nanofiber carries 0.01 - 50 mg of vitronectin per 1 g of the chitin nanofiber.

3. The method according to claim 1 or claim 2, wherein a chitosan nanofiber is further added in step (ii).

4. The method according to claim 3, wherein a content ratio (weight) of the chitin nanofiber carrying vitronectin and the chitosan nanofiber is chitin nanofiber carrying vitronectin :chitosan nanofiber = 1:0.5 - 20.

5. A composition for addition to a medium, comprising a chitin nanofiber carrying vitronectin and a chitosan nanofiber, wherein carrying is a state in which the nanofiber and vitronectin are attached or adsorbed without a chemical covalent bond.

6. The composition according to claim 5, wherein the chitin nanofiber carries 0.01 - 50 mg of vitronectin per 1 g of the chitin nanofiber.

7. The composition according to claim 5 or 6, wherein a content ratio (weight) of the chitin nanofiber carrying vitronectin and the chitosan nanofiber contained in medium composition is chitin nanofiber carrying vitronectin :chitosan nanofiber = 1:0.5 - 20.

8. A medium composition for suspension culture of an adherent cell, comprising the composition according to any one of claims 5 to 7.

## Patentansprüche

1. Verfahren zur Herstellung einer Medium-Zusammensetzung für die Suspensionskultur einer adhärenten Zelle, umfassend die folgenden Schritte:
(i) einen Schritt der Herstellung von Vitronectin, das auf einer aus Chitin bestehenden Nanofaser getragen wird,
(ii) einen Schritt der Zugabe der in Schritt (i) erhaltenen Vitronectin-tragenden Chitin-Nanofaser zu einem Medium,
wobei getragen ein Zustand ist, in dem die Nanofaser und Vitronectin ohne eine chemische kovalente Bindung gebunden oder adsorbiert sind.

2. Verfahren nach Anspruch 1, wobei die Chitin-Nanofaser 0,01 - 50 mg Vitronectin pro 1 g der Chitin-Nanofaser trägt.

3. Verfahren nach Anspruch 1 oder Anspruch 2, wobei in Schritt (ii) zusätzlich eine Chitosan-Nanofaser zugegeben wird.

4. Verfahren nach Anspruch 3, wobei das Gehaltsverhältnis (Gewicht) der Vitronectin-tragenden Chitin-Nanofaser und der Chitosan-Nanofaser 1 : 0,5 - 20 beträgt.

5. Zusammensetzung zur Zugabe zu einem Medium, umfassend eine Vitronectin-tragende Chitin-Nanofaser und eine Chitosan-Nanofaser, wobei tragend ein Zustand ist, in dem die Nanofaser und Vitronectin ohne eine chemische kovalente Bindung gebunden oder adsorbiert sind.

6. Zusammensetzung nach Anspruch 5, wobei die Chitin-Nanofaser 0,01 - 50 mg Vitronectin pro 1 g der Chitin-Nanofaser trägt.

7. Zusammensetzung nach Anspruch 5 oder 6, wobei das Gehaltsverhältnis (Gewicht) der Vitronectin-tragenden Chitin-Nanofaser und der in der Medium-Zusammensetzung enthaltenen Chitosan-Nanofaser, Vitronectin tragende Chitin-Nanofaser : Chitosan-Nanofaser = 1 : 0,5 - 20 ist.

8. Medium-Zusammensetzung für die Suspensionskultur einer adhärenten Zelle, umfassend die Zusammensetzung nach einem der Ansprüche 5 bis 7.

## Revendications

1. Procédé pour produire une composition de milieu pour culture en suspension d'une cellule adhérente, comprenant les étapes suivantes :
(i) une étape de préparation de vitronectine supportée sur des nanofibres composées de chitine,
(ii) une étape d'addition des nanofibres de chitine supportant de la vitronectine obtenues dans l'étape (i) à un milieu,
dans lequel l'état supporté est un état dans lequel les nanofibres et la vitronectine sont attachées ou adsorbées sans liaison covalente chimique.

2. Procédé selon la revendication 1, dans lequel les nanofibres de chitine supportent 0,01 à 50 mg de vitronectine par gramme des nanofibres de chitine.

3. Procédé selon la revendication 1 ou la revendication 2, dans lequel des nanofibres de chitosane sont en outre ajoutées à l'étape (ii).

4. Procédé selon la revendication 3, dans lequel le rapport en teneur (poids) des nanofibres de chitine supportant la vitronectine aux nanofibres de chitosane est (nanofibres de chitine supportant de la vitronectine / nanofibres de chitosane) = 1 / 0,5 à 20.

5. Composition pour addition à un milieu, comprenant des nanofibres de chitine supportant de la vitronectine et des nanofibres de chitosane, dans laquelle l'état supporté est un état dans lequel les nanofibres et la vitronectine sont attachées ou adsorbées sans liaison covalente chimique.

6. Composition selon la revendication 5, dans laquelle les nanofibres de chitine supportent 0,01 à 50 mg de vitronectine par gramme des nanofibres de chitine.

7. Composition selon la revendication 5 ou 6, dans laquelle le rapport en teneur (poids) des nanofibres de chitine supportant la vitronectine aux nanofibres de chitosane contenues dans la composition de milieu est (nanofibres de chitine supportant de la vitronectine / nanofibres de chitosane) = 1 / 0,5 à 20.

8. Composition de milieu pour culture en suspension d'une cellule adhérente, comprenant la composition selon l'une quelconque des revendications 5 à 7.
